(19) Europäisches Patentamt
European Patent Office
Office européen des brevets

(11) EP 0 856 511 B1

(12) **FASCICULE DE BREVET EUROPEEN**

(45) Date de publication et mention de la délivrance du brevet:
**03.03.2004 Bulletin 2004/10**

(51) Int Cl.[7]: **C07D 209/28**, A61K 31/405

(21) Numéro de dépôt: **98400170.1**

(22) Date de dépôt: **28.01.1998**

(54) **Indométacine calcique pentahydratée et sa préparation**

Indometacin Calciumsalz Pentahydrat und deren Herstellung

Calcium salt of indomethacin pentahydrate and its preparation

(84) Etats contractants désignés:
**AT BE CH DE DK ES FI FR GB GR IE IT LI LU MC NL PT SE**

(30) Priorité: **29.01.1997 FR 9700925**

(43) Date de publication de la demande:
**05.08.1998 Bulletin 1998/32**

(73) Titulaire: **Promindus (Actions Promotionnelles dans l'Industrie et le Commerce)**
**21000 Casablanca (MA)**

(72) Inventeurs:
- **Bennis, Abderrahim**
  **Casablanca (MA)**
- **Serrano, Jean-Jacques**
  **31090 Montpellier (FR)**
- **Bennis, Farid**
  **Casablanca (MA)**

(74) Mandataire: **Le Roux, Martine et al**
**Cabinet Beau de Loménie**
**158, rue de l'Université**
**75340 Paris Cédex 07 (FR)**

(56) Documents cités:
**EP-A- 0 006 223** **EP-A- 0 245 126**

- **CHEMICAL ABSTRACTS, vol. 110, no. 14, 3 avril 1989 Columbus, Ohio, US; abstract no. 127438u, WU,JIGUI ET AL: "Synthesis of metal salts of 1-p-chlorobenzoyl-2-methyl-5-methoxyindol-3-acetic acid" XP002041946 & LANZHOU DAXUE XUEBAO,ZIRAN KEXUEBAN, vol. 24, no. 2, - 1988 pages 74-77,**
- **CHEMICAL ABSTRACTS, vol. 101, no. 20, 12 novembre 1984 Columbus, Ohio, US; abstract no. 177352w, OGISO,TARO ET AL: "Absorption and bioavailability of calcium and magnesium salts of indomethacin ..." XP002041947 & J. PHARMACOBIO-DYN, vol. 7 6, - 1988 pages 392-399,**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 856 511 B1

## Description

La présente invention a pour objets :

- un nouveau sel de l'indométacine,
- des compositions pharmaceutiques, renfermant, à titre de principe actif, ledit nouveau sel de l'indométacine ;
- un procédé de préparation dudit nouveau sel

L'indométacine ou acide 1-(p-chlorobenzoyl)-5-méthoxy-2-méthylindole-3-acétique, répondant à la formule ci-après :

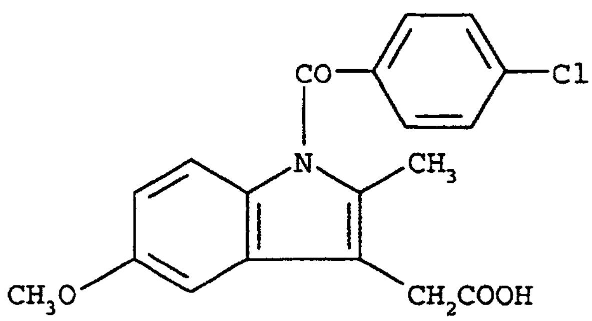

est un composé bien connu, depuis le début des années 1960, qui présente des propriétés pharmacologiques intéressantes. Un procédé de synthèse dudit composé a notamment été décrit dans le brevet US-A-3,161,654. Ledit composé est proposé, depuis de nombreuses années, comme anti-inflammatoire. Il est inhibiteur des prostaglandines. Il présente toutefois un inconvénient majeur : sa gastro-toxicité.

De nombreux documents de l'art antérieur sont relatifs à des sels de l'indométacine. En particulier :

- le Chemical Abstrat, Vol. 110, 1989, page 894, n° 127438u fait état d'une étude chimique sur des sels hydratés de l'indométacine avec des métaux divalents, sels de formule :

$$M(Ind)_2.n\ H_2O$$

dans laquelle

$$M = Mg, Ca, Cr, Ba, Mn, Co, Ni, Cu, Zn, Pb \text{ et } n = 1 - 4 ;$$

- l'article du J. Pharm. Dyn., 7, 392-399(1984) présente une étude comparative de l'absorption et de la disponibilité des sels de calcium et de magnésium de l'indométacine, administrés par voie rectale ;
- la demande de brevet EP-A-6223 concerne les sels de sodium et de potassium de l'indométacine ainsi que les formes trihydratées respectives ;
- la demande de brevet EP-A-245126 décrit des préparations dermiques contenant les sels de calcium de produits anti-inflammatoires, tel que l'indométacine.

La Demanderesse a montré, de façon surprenante, qu'un sel original spécifique de l'indométacine - sel de calcium pentahydraté - présente, par rapport à la forme acide, tout en conservant une activité pharmacologique équivalente :

- par voie orale, une toxicité diminuée;
- par voie rectale, une meilleure tolérance locale.

De tels résultats sont tout à fait surprenants dans la mesure où l'on pouvait s'attendre à l'hydrolyse in vivo dudit sel de calcium (comme tel est le cas avec les sels de magnésium et de lithium, par exemple) et donc à la manifestation de la toxicité de l'indométacine acide.

Ledit sel de calcium pentahydraté de l'indométacine ou indométacine calcique pentahydratée constitue le premier objet de la présente invention.

L'amélioration remarquable du couple efficacité/tolérance de ce sel a conduit la Demanderesse à en développer un procédé de fabrication, particulièrement performant, qui constitue un autre objet de ladite présente invention.

Selon son premier objet, la présente invention concerne donc l'indométacine calcique pentahydratée qui répond :

- à la formule brute, ci-après : $C_{38}H_{30}Cl_2N_2O_8Ca,5H_2O$ ;
- à la formule développée, ci-après :

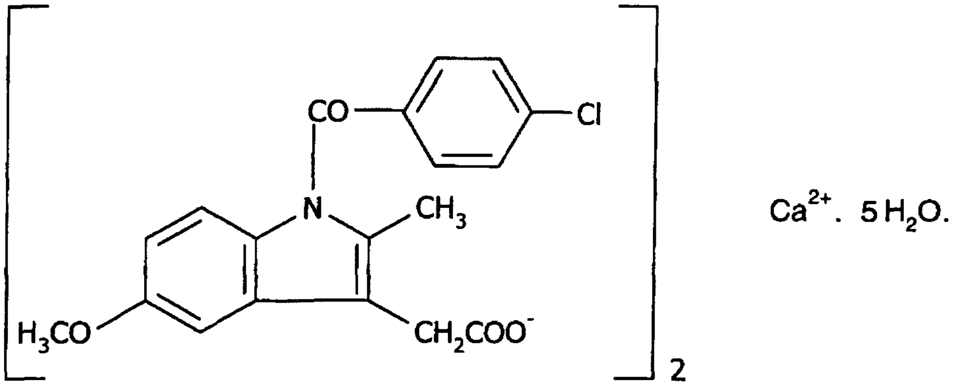

La masse relative de ce nouveau composé est $M_r$ = 843,68.

L'indométacine calcique pentahydratée se présente sous la forme d'une poudre cristalline jaune, inodore ou sensiblement inodore, pratiquement insoluble dans l'eau, dans le chloroforme, dans l'éther et dans l'acétonitrile, facilement soluble dans le diméthylformamide, soluble dans le tétrahydrofuranne.

Le point de fusion, avec décomposition, de l'indométacine calcique pentahydratée est voisin de 240°C (celui de l'indométacine (acide) est : 158 à 162°C).

Dans le cadre de son premier objet, la présente invention concerne plus particulièrement ladite indométacine calcique, renfermant une teneur inférieure ou égale à 0,5 % en poids d'indométacine libre (acide) et/ou une teneur inférieure ou égale à 0,2 % en poids de produit(s) de dégradation de l'indométacine. Il s'agit là de formes préférées du produit de l'invention. Des formes particulièrement préférées sont celles qui renferment, à la fois :

- une teneur en indométacine libre inférieure ou égale à 0,5 % en poids ; et
- une teneur en produit(s) de dégradation de l'indométacine inférieure ou égale à 0,2 % en poids.

Lesdites formes préférées et particulièrement préférées peuvent être obtenues directement à l'issue de variantes optimisées de mise en oeuvre du procédé de l'invention ou à l'issue d'une étape complémentaire de purification faisant suite à une mise en oeuvre non optimisée du procédé de l'invention.

On précise ici, à toutes fins utiles, que les principaux produits de dégradation de l'indométacine consistent en l'acide parachlorobenzoïque et en l'acide 5-méthoxy-2-méthyl-3-indole acétique. Ceci est bien connu de l'homme du métier.

Comme indiqué ci-dessus, l'indométacine calcique (pentahydratée) selon l'invention conserve toutes les propriétés pharmacologiques de l'indométacine (libre). Elle peut donc être utilisée dans les mêmes indications thérapeutiques. De surcroît, elle offre l'avantage de présenter une toxicité plus faible et une meilleure tolérance que ladite indométacine (libre).

En effet, les comparaisons respectives des $DE_{50}$ (dose efficace 50 %) et $DL_{50}$ (dose léthale 50 %) *per os*, chez le rat, de ladite indométacine calcique pentahydratée et de ladite indométacine libre sont nettement à l'avantage de ladite indométacine calcique.

On a indiqué les résultats obtenus, dans le tableau ci-après :

| | Indométacine (mg/kg) Art antérieur | Indométacine calcique pentahydratée (mg/kg) Invention |
|---|---|---|
| $DE_{50}$ | 3 | 2,5 |
| $DL_{50}$ | 28 | 45 |
| Index thérapeutique : $\frac{DL_{50}}{DE_{50}}$ | 9,3 | 18 |

Selon son second objet, la présente invention concerne donc logiquement des compositions pharmaceutiques ou médicaments qui contiennent une quantité pharmacologiquement (thérapeutiquement) active d'indométacine calcique pentahydratée. Ladite indométacine calcique pentahydratée - principe actif- est avantageusement incorporée dans un excipient, support ou véhicule pharmaceutiquement acceptable. Lesdites compositions pharmaceutiques de l'invention peuvent notamment être formulées comme dcs préparations galéniques convenant pour l'administration par voie orale, par voie rectale, par voie injectable ou pour des applications locales, dermiques ou ophtalmiques.

Il est clair que l'indométacine calcique pentahydratée de l'invention se substitue avantageusement à l'indométacine acide (libre) de l'art antérieur, dans toutes les indications thérapeutiques de cette dernière ; indications thérapeutiques familières à l'homme du métier.

Selon son troisième objet, la présente invention concerne un procédé pour la préparation de ladite indométacine calcique pentahydratée.

Ledit procédé comprend la salification de l'indométacine (indométacine acide-libre) par le carbonate de calcium ; réaction de salification mise en oeuvre, selon la réaction :

$$2 \text{ Indométacine} + Ca\,CO_3 \rightarrow (\text{Indom.})_2\,Ca + CO_2$$

dans les conditions ci-après :

- en milieu aqueux : l'homme du métier comprend aisément l'intérêt qu'il y a à faire intervenir l'eau à titre de solvant (pour des raisons notamment économiques, écologiques, de neutralité réactionnelle, de non-toxicité et de pureté du produit attendu) ;
- dans une proportion molaire des réactifs indométacine (acide) : carbonate de calcium comprise entre 2 : 1 et 2 : 2 : on fait intervenir le carbonate de calcium au minimum en la quantité stoechiométrique voire en des quantités supérieures à celle-ci, de façon à salifier au maximum l'acide (indométacine libre) et donc à minimiser la présence dudit acide dans le produit final. Si la quantité d'intervention dudit carbonate de calcium est limitée à ladite quantité stoechiométrique, on se dispense, de la manipulation, par la suite, de l'excès de carbonate de calcium. On minimise aussi le risque de formation des produits de dégradation de l'indométacine, dans la mesure où ladite formation est favorisée en milieu alcalin. Si ledit carbonate de calcium intervient en une quantité supérieure à ladite quantité stoechiométrique, on obtient l'indométacine calcique avec l'excès dudit carbonate de calcium (par rapport à la proportion stoechiométrique). Cet excès est positif dans la mesure où il permet une meilleure gestion du procédé (rendu moins sensible aux variations des paramètres-clés de la réaction) et négatif pour la raison indiquée ci-dessus. Un compromis raisonnable s'impose. En fin de réaction, l'excès de $CaCO_3$ n'est pas outre mesure gênant car ledit carbonate de calcium peut notamment convenir comme excipient pharmaceutiquement acceptable pour la formulation de l'indométacine calcique préparée.

Les réactifs indométacine (acide): carbonate de calcium interviennent avantageusement dans une proportion molaire comprise entre 2: 1 et 2: 1,5. De façon particulièrement préférée, il interviennent dans une proportion molaire

- correspondante à la stoechiométrie : 2 : 1,
  ou
- proche de ladite stoechiométrie, et notamment correspondante à la proportion molaire : 2 : 1,1.

Avec les réactifs intervenants en proportion stoechiométrique (2 :1), on a obtenu de très bons résultats tant

en termes de rendement qu'en termes de pureté du sel obtenu ; avec un léger excès de carbonate de calcium (notamment : 2 : 1,1), on peut encore obtenir de bons, voire de très bons résultats en termes de rendement et pureté du sel obtenu et ceci avec une mise en oeuvre plus souple du procédé, ce qui peut être appréciable.

Au cours de sa formation, l'indométacine calcique se présente sous la forme d'une pâte jaune, qui est recueillie puis séchée, en étuve ventilée, notamment dans les conditions ci-après : à 60°C, pendant 12 heures. La poudre jaune pâle sèche obtenue est ensuite laissée à l'air ambiant jusqu'à ce que sa masse demeure constante. On obtient ainsi le sel pentahydraté.

A l'issue de la réaction de salification, comme indiqué ci-dessus, on récupère la poudre d'indométacine calcique pentahydratée. Ladite poudre est, si nécessaire, purifiée, par lavage avec des solvants organiques convenables (tel le chloroforme) afin de diminuer voire d'éliminer, autant que faire se peut, l'indométacine libre et le(s) produit(s) de dégradation de l'indométacine qu'elle renferme.

On vise avantageusement, dans le cadre de la présente invention, à obtenir les formes préférées et particulièrement préférées de l'indométacine calcique pentahydratée; à savoir des formes, qui renferment :

- une teneur en indométacine libre, inférieure ou égale à 0,5 % en poids, et/ou (avantageusement et)
- une teneur en produit(s) de dégradation, inférieure ou égale à 0,2 % en poids.

Pour atteindre ce but, il peut s'avérer nécessaire, comme indiqué ci-dessus, de faire suivre l'étape de salification (mise en oeuvre dans des conditions non optimisées) d'une étape de purification mais, selon une variante avantageuse du procédé de l'invention, on peut obtenir directement du produit répondant aux critères de pureté ci-dessus. Il convient, à cet effet, de mettre en oeuvre la salification dans des conditions contrôlées adéquates.

La mise en oeuvre du procédé de l'invention, dans de telles conditions, explicitées ci-après, constitue une variante particulièrement préférée du procédé revendiqué.

Selon ladite variante préférée, les réactifs indométacine (libre) et carbonate de calcium ($CaCO_3$) sont mis à réagir :

- <u>à une température inférieure ou égale à 30°C</u> : on préconise de mettre en oeuvre la salification par $CaCO_3$ à une température comprise entre 15 et 30°C, avantageusement à une température comprise entre 20 et 25°C. La température de mise en oeuvre de la réaction correspond à un compromis entre la vitesse de ladite réaction et le risque de formation de(s) produit(s) de dégradation de l'indométacine. On préconise vivement de maintenir le réacteur en deçà de 30°C, pour assurer, dans le mélange réactionnel final, une teneur en produit(s) de dégradation inférieure à 0,2 % en poids ;
- <u>dans des conditions où l'accumulation, dans le milieu réactionnel, du $CO_2$ formé est minimisée, voire annulée</u> : la salification est en effet quantitative à la condition d'agiter suffisamment le mélange réactionnel et d'éviter, dans celui-ci, l'accumulation du gaz carbonique. Dans une telle réaction, l'agitation du mélange réactionnel assure, d'une part, de façon classique, l'homogénéité de la répartition des produits réactifs et elle a, d'autre part, pour effet de favoriser l'élimination du $CO_2$ généré par la réaction (cette élimination est bénéfique tant du point de vue de l'équilibre chimique que du fait qu'elle limite voire évite la formation de produits parasites type bicarbonate ...). Le procédé de l'invention est donc, dans le cadre de cette variante avantageuse, mis en oeuvre sous agitation efficace.

Outre l'agitation, pour le dégazage du $CO_2$ formé , on peut mettre avantageusement en oeuvre; indépendamment, voire conjointement; les deux techniques ci-après :

- soumission du réacteur à un vide intermittent ou continu :de manière générale, dans les minutes qui suivent l'addition du $CaCO_3$, la production du $CO_2$ est importante et tend à faire mousser le mélange réactionnel. On a donc jugé opportun d'appliquer un vide, piloté, de façon intermittente, en fonction du niveau de moussage, en ce début de réaction. Ledit vide se situe généralement entre 0,1 et 0,5 $10^5$ Pa (0,1 et 0,5 bar). Après cette phase initiale de moussage (qui peut se répéter si le $CaCO_3$ est ajouté de façon fractionnée (voir ci-après)), le vide appliqué est généralement maintenu entre 0,2 et 1 $10^5$ Pa (0,2 et 1 bar), de préférence entre 0,5 et 0,7 $10^5$ Pa (0,5 et 0,7 bar). Selon le type de réacteur (mélangeur) utilisé, un vide plus poussé peut être appliqué ;
- l'introduction, dans ledit réacteur, du réactif $CaCO_3$, de façon fractionnée (de façon à limiter ainsi la concentration du $CO_2$ dans le mélange réactionnel).

Pour obtenir le premier effet escompté par l'agitation -à savoir une homogénéisation suffisante- on veille à faire intervenir selon le type de réacteur (mélangeur) et selon les quantités mises en oeuvre, les quantités adéquates de solvant (eau). Ledit solvant (eau) intervient avantageusement dans les quantités ci-après : la proportion d'eau, exprimée en litres, du mélange réactionnel est comprise entre 2 et 10 fois, de préférence entre 2 et 7 fois, le poids d'indométacine libre (réactif), exprimé en kilogrammes.

A l'issue de la mise en oeuvre, plus ou moins optimisée, du procédé de l'invention, on rappelle que l'on obtient une pâte qu'il convient généralement de sécher (notamment à l'étuve) puis de laisser, à l'air ambiant, jusqu'à ce que sa masse demeure constante.

Comme le lecteur l'a compris, ladite pâte peut éventuellement renfermer, outre le produit attendu (l'indométacine calcique pentahydratée), du carbonate de calcium (réactif en excès), de l'indométacine libre (avantageusement moins de 0,5 % en poids), des produits de dégradation de l'indométacine (avantageusement moins de 0,2 % en poids).

Le procédé de l'invention décrit ci-dessus est particulièrement intéressant en ce que :

- il peut être mis en oeuvre à différentes échelles de quantités (laboratoire, pilote, production industrielle) ; son extrapolation ne soulève aucune difficulté particulière ;
- il peut être mis en oeuvre dans différents types de réacteurs (mélangeurs) ;
- il offre des avantages, qui ne seraient pas obtenus ensemble, ni avec d'autres dérivés du calcium (tels que, par exemple, l'hydroxyde de calcium ou le glycérophosphate de calcium), ni avec d'autres cations alcalino ou alcalino-terreux ; à savoir :

  + une absence de produits tiers, résiduels, dans la mesure où le gaz carbonique ($CO_2$) peut être aisément éliminé ;
  + une mise en oeuvre d'un nombre réduit de réactifs : l'indométacine (libre), le carbonate de calcium ($CaCO_3$), le solvant (eau) ;
  + des conditions de réalisation et de prix de revient, compatibles avec une exploitation industrielle ;
  + l'obtention (avantageusement directe) d'un produit dont la qualité, la stabilité, l'activité et la tolérance sont compatibles avec une utilisation pharmaceutique.

L'homme du métier, à la lecture de ce qui précède, a déjà saisi tout l'intérêt de la présente invention, qui propose à la fois :

- une forme originale d'un principe actif de l'art antérieur ; forme originale qui a conservé les propriétés thérapeutiques dudit principe actif de l'art antérieur et ne présente plus sa toxicité ;
- un procédé, simple et performant, pour la préparation de ladite forme originale, tant à l'échelle du laboratoire, du pilote qu'à l'échelle industrielle.

Selon son dernier objet, la présente invention concerne l'indométacine calcique susceptible d'être obtenue directement par les variantes avantageusement optimisées du procédé de l'invention et renfermant donc une teneur inférieure ou égale à 0,5 % en poids d'indométacine libre et/ou une teneur inférieure ou égale à 0,2 % en poids de produit(s) de dégradation de l'indométacine.

L'invention présentement revendiquée est illustrée par les exemples ci-après. Elle est plus précisément illustrée par les résultats donnés dans le tableau 1 ci-après (lots B, D à K).

La Demanderesse a mis en oeuvre, dans différentes conditions, le procédé de l'invention, pour obtenir, à différents rendements et degrés de pureté, l'indométacine calcique pentahydratée.

Ledit procédé de l'invention a été mis en oeuvre selon diverses variantes, tant à l'échelle du laboratoire (réacteur de 1 l ou de 5 l) qu'à l'échelle pilote (réacteur de 100 l). Quelle que soit la variante de mise en oeuvre, on a introduit , dés le départ, les réactifs (indométacine libre + $CaCO_3$), en milieu aqueux, en différentes proportions, dans le réacteur utilisé.

Ledit réacteur utilisé, de 1 l ou de 5 l (échelle laboratoire) ou de 100 l (échelle pilote) consiste en un mélangeur soit du type mélangeur à palettes, soit du type mélangeur pour produit pâteux (plus précisément, réacteur MOLTOMAT équipé de son système d'agitation ).

a) La synthèse de l'indométacine calcique a été mise en oeuvre dans des mélangeurs à palettes :

- de 100 l, pour la production du lot A;
- de 1 l, pour la production des lots B et C;

dans les conditions ci-après :

- à une température comprise entre 25 et 30°C ;
- à la pression atmosphérique ;
- en milieu aqueux, dans les proportions ci-après :

volume d'eau (en litres)/poids d'indométacine (en kg) = 10;

- avec différents rapports molaires des réactifs : indométacine : $CaCO_3$.

Les résultats obtenus, quant à la pureté de l'indométacine produite, figurent dans la première partie du tableau 1 ci-après.

b) La synthèse de l'indométacine calcique a été mise en oeuvre dans des mélangeurs pour produit pâteux (réacteurs MOLTOMAT) :

- de 100 l, pour la production des lots D, G et H ;
- de 5 l, pour la production des lots E, F, I, J, K ; dans les conditions ci-après :
  - sous un vide de 0,1 - 0,5 bar (lots F, I, J, K); à la pression atmosphérique (lots D, E, G, H)
  - à une température comprise entre 25 et 30°C, pour les lots I, J, K et sans contrôle de ladite température pour les lots D, E, F, G, H, (température qui est montée jusqu'à 50-60°C, pour lesdits lots D, E et F);
- en milieux aqueux; dans les proportions ci-après :
  + soit volume d'eau (en litres) / poids d'indométacine (en kg) = 10
  + soit volume d'eau (en litres) / poids d'indométacine (en kg) compris entre 3 et 7 ;
- avec différents rapports molaires des réactifs : indométacine : $CaCO_3$.

Les résultats obtenus, quant à la pureté de l'indométacine produite, figurent dans la seconde partie du tableau 1 ci-après.

Tableau 1

| **Proportions Indométacine : $CaCO_3$** | **Indométacine libre (% en poids sur sec)** | **Produits de dégradations*** (ac. MMIA+ac.p.CB) (% en poids sur sec)** |
|---|---|---|
| En mélangeur à palettes* | | |
| 2 : 3,57 (lot A) | 0,12 | 0,25 |
| 2 : 1 (stoechiom.)(lot B) | 0,12 | - |
| 2 : 0,5 (lot C) | 45,7<br>↑<br>(ce qui correspond à ≅ 50 % d'indométacine combiné sous forme d'indométacine calcique) | - |
| En mélangeur pour produit pâteux | | |
| - sans refroidissement | | |
| 2 : 1*(stoechiom.)(lot D) | 7 | - |
| 2 : 1*(stoechiom.)(lot E) | 3 | - |
| 2 : 1**(stoechiom.)(lot F) | 0,15 | 0,27 |
| 2 : 1,1*(lot G) | 0,12 | 0,06 |
| 2 : 1,5*(lot H) | 0,16 | 0,03 |
| - avec refroidissement** | | |
| 2 : 1 (stoechiom.)(lot I) | 0,19 | 0,13 |

* Volume d'eau (en litres) / poids d'indométacine (en kg) = 10

** Volume d'eau (en litres) / poids d'indométacine (en kg) : > 3 et < 7

*** Ac.MMIA = acide 5-méthoxy-2-méthyl-3-indole acétique ;
Ac.p-CB = acide parachlorobenzoïque

Tableau 1 (suite)

| Proportions Indométacine : $CaCO_3$ | Indométacine libre (% en poids sur sec) | Produits de dégradations*** (ac. MMIA+ac.p.CB) (% en poids sur sec) |
|---|---|---|
| - avec refroidissement** | | |
| 2 : 1 (stoechiom.)(lot J) | 0,15 | 0,07 |
| 2 : 1 (stoechiom.)(lot K) | 0,11 | 0,11 |

** Volume d'eau (en litres) / poids d'indométacine (en kg) : > 3 et < 7

*** Ac.MMIA = acide 5-méthoxy-2-méthyl-3-indole acétique ;
Ac.p-CB = acide parachlorobenzoïque

La considération desdits résultats appelle les commentaires ci-après :

- la salification, selon l'invention, de l'indométacine est quantitative ;
- le taux pondéral d'indométacine libre dans le produit obtenu (indométacine calcique pentahydratée) est ≤ 0,5 % pour les proportions stoechiométriques (lots B, F, I, J, K). Des excès de $CaCO_3$ de 357 % (lot A), de 50 % (lot H), de 10 % (lot G) permettent aussi d'obtenir l'indométacine calcique avec un taux pondéral d'indométacine libre ≤ 0,5 %. Avec les proportions indométacine : $CaCO_3$ de 2 : 0,5 , le taux pondéral d'indométacine libre dans le produit avoisine les 50 % ;
- lorsque l'agitation n'est pas suffisante ou en absence de vide (lots D et E), i.e. lorsque le $CO_2$ n'est pas suffisamment éliminé, le taux d'indométacine libre augmente ; (ce taux pourrait être diminué en augmentant la proportion de $CaCO_3$);
- lorsque la température du milieu réactionnel dépasse 30°C (ces 30°C étant d'autant plus vite atteints que le volume d'eau est faible (voir le lot F)), on favorise l'apparition des produits de dégradation ; leur taux pondéral devient supérieur à 0,2 % (lot F). Le refroidissement du réacteur, de façon à maintenir ladite température du milieu réactionnel inférieure ou égale à 30°C, permet d'obtenir un produit très pur ;
- les résultats, obtenus pour les lots G et H, en l'absence de refroidissement et de vide mais avec une agitation suffisante, sont bons, dans la mesure où la proportion de $CaCO_3$ est supérieure à celle du mélange réactionnel stoechiométrique.

Le procédé de l'invention, selon ses variantes optimisées, permet d'obtenir directement, à l'échelle industrielle, l'indométacine calcique pentahydratée :

- avec un taux d'indométacine libre inférieur ou égal à 0,5 %, en poids ;
- avec un taux de produit(s) de dégradation inférieur ou égal à 0,2 % en poids.

En cela, le procédé de l'invention est particulièrement intéressant.

## Revendications

1. Indométacine calcique pentahydratée, répondant à la formule développée ci-après :

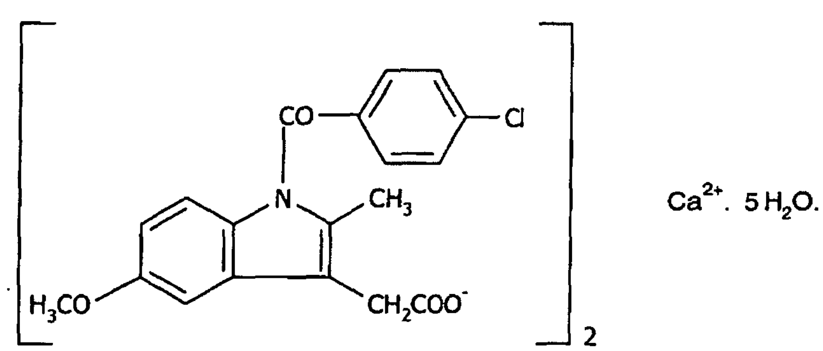

2. Indométacine calcique selon la revendication 1, **caractérisée en ce qu'**elle renferme une teneur inférieure ou égale à 0,5 % en poids d'indométacine libre et/ou une teneur inférieure ou égale à 0,2 % en poids de produit(s) de dégradation de l'indométacine.

3. Compositions pharmaceutiques, **caractérisées en ce qu'**elles contiennent une quantité pharmacologiquement active d'indométacine calcique selon l'une des revendications 1 ou 2, avantageusement incorporée dans un excipient, support ou véhicule pharmaceutiquement acceptable.

4. Compositions pharmaceutiques selon la revendication 3, **caractérisées en ce qu'**elles consistent en des préparations galéniques acceptables pour l'administration par voie orale, par voie rectale, par voie injectable ou pour des applications locales, dermiques ou ophtalmiques.

5. Procédé pour la préparation d'indométacine calcique pentahydratée **caractérisé en ce qu'**il comprend : - la réaction, en milieu aqueux, de l'indométacine (acide) avec du carbonate de calcium; lesdits réactifs indométacine (acide) ; carbonate de calcium intervenant dans une proportion molaire comprise entre 2 : 1 et 2 : 2 ;

   - le séchage de la pâte obtenue à l'issue de ladite réaction puis son maintien à l'air ambiant jusqu'à ce que sa masse demeure constante.

6. Procédé selon la revendication 5, **caractérisé en ce que** lesdits réactifs interviennent dans une proportion molaire, stoechiométrique (2 : 1) ou proche de la stoechiométrie.

7. Procédé selon l'une des revendications 5 ou 6, **caractérisé en ce que** lesdits réactifs interviennent dans une proportion molaire de 2 : 1,1.

8. Procédé selon l'une quelconque des revendications 5 à 7, **caractérisé en ce que** ladite réaction est mise en oeuvre :

   - à une température inférieure ou égale à 30°C ;
   - dans des conditions où l'accumulation, dans le milieu réactionnel, du $CO_2$ formé est minimisée, voire annulée.

9. Procédé selon l'une quelconque des revendications 5 à 8, **caractérisé en ce que** ladite réaction est mise en oeuvre à une température inférieure à 30°C.

10. Procédé selon l'une quelconque des revendications 5 à 9, **caractérisé en ce qu'**il est mis en oeuvre sous agitation.

11. Procédé selon la revendication 10, **caractérisé en ce qu'**il est mis en oeuvre avec application d'un vide intermittent ou continu et/ou introduction du carbonate de calcium dans le mélange réactionnel de façon fractionnée.

12. Procédé selon l'une quelconque des revendications 5 à 11, **caractérisé en ce que** la proportion d'eau, exprimée en litres, du mélange réactionnel est comprise entre 2 et 10 fois le poids d'indométacine (libre), exprimé en kilogrammes.

13. Procédé selon l'une quelconque des revendications 5 à 12, **caractérisé en ce que** la proportion d'eau, exprimée en litres, du mélange réactionnel est comprise entre 2 et 7 fois le poids d'indométacine (libre), exprimée en kilogrammes.

**Patentansprüche**

1. Indometacin-calcium-pentahydrat der Form

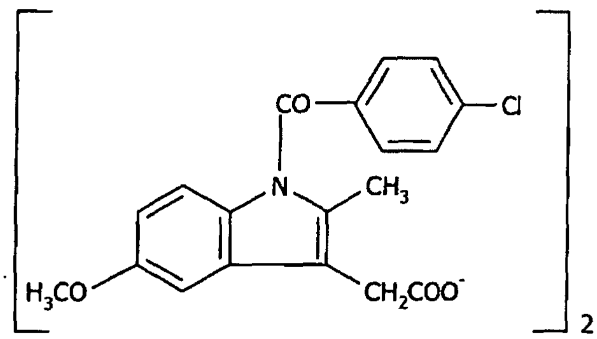

$Ca^{2+} . 5 H_2O$.

**2.** Indometacin-calcium nach Anspruch 1, **dadurch gekennzeichnet, dass** es ≤ 0,5 Gew.-% freies Indometacin und/oder ≤ 0,2 Gew.-% Indometacin-Abbauprodukt(e) enthält.

**3.** Pharmazeutische Zusammensetzungen, **dadurch gekennzeichnet, dass** sie eine pharmakologisch aktive Menge Indometacin-calcium nach einem der Ansprüche 1 oder 2 enthalten, das vorteilhaft einem pharmazeutisch akzeptablen Exzipienten, Träger oder Vehiculum einverleibt ist.

**4.** Pharmazeutische Zusammensetzungen nach Anspruch 3, **dadurch gekennzeichnet, dass** sie aus galenischen Präparaten bestehen, die geeignet sind für die Verabreichung auf oralem, rektalem oder injizierbarem Wege oder für lokale dermale oder ophthalmische Aufträge.

**5.** Verfahren zur Herstellung von Indometacin-calcium-pentahydrat, **dadurch gekennzeichnet, dass** es umfasst:

- die Umsetzung von Indometacin (Säure) mit Calciumcarbonat in einem wässrigen Medium, wobei die Reagentien Indometacin (Säure) und Calciumcarbonat in einem Molverhältnis zwischen 2 : 1 und 2 : 2 eingesetzt werden;
- die Trocknung der bei der oben genannten Reaktion erhaltenen Paste und das anschließende Halten derselben in der umgebenden Luft bis die Masse konstant bleibt.

**6.** Verfahren nach Anspruch 5, **dadurch gekennzeichnet, dass** die Reagentien in einem stöchiometrischen Molverhältnis (2 : 1) oder in einem nahezu stöchiometrischen Molverhältnis eingesetzt werden.

**7.** Verfahren nach einem der Ansprüche 5 oder 6, **dadurch gekennzeichnet, dass** die Reagentien in einem Molverhältnis von 2 : 1,1 eingesetzt werden.

**8.** Verfahren nach einem der Ansprüche 5 bis 7, **dadurch gekennzeichnet, dass** die Reaktion durchgeführt wird:

- bei einer Temperatur von ≤ 30 °C,
- unter solchen Bedingungen, bei denen die Anreicherung des gebildeten $CO_2$ in dem Reaktionsmedium minimiert oder sogar vermieden wird.

**9.** Verfahren nach einem der Ansprüche 5 bis 8, **dadurch gekennzeichnet, dass** die Reaktion bei einer Temperatur unterhalb 30 °C durchgeführt wird.

**10.** Verfahren nach einem der Ansprüche 5 bis 9, **dadurch gekennzeichnet, dass** es unter Rühren durchgeführt wird.

**11.** Verfahren nach Anspruch 10, **dadurch gekennzeichnet, dass** es unter Anlegen eines intermittierenden oder kontinuierlichen Vakuums und/oder unter fraktionierter Einführung von Calciumcarbonat in die Reaktionsmischung durchgeführt wird.

**12.** Verfahren nach einem der Ansprüche 5 bis 11, **dadurch gekennzeichnet, dass** der Mengenanteil an Wasser, ausgedrückt in Liter, der Reaktionsmischung zwischen dem 2- und dem 10-fachen des Gewichtes an (freiem) Indometacin, ausgedrückt in kg, liegt.

**13.** Verfahren nach einem der Ansprüche 5 bis 12, **dadurch gekennzeichnet, dass** der Mengenanteil an Wasser, ausgedrückt in Liter, der Reaktionsmischung zwischen dem 2- und dem 7-fachen des Gewichtes an (freiem) Indometacin, ausgedrückt in kg, liegt.

**Claims**

**1.** Indomethacin calcium pentahydrate, having the formula shown below :

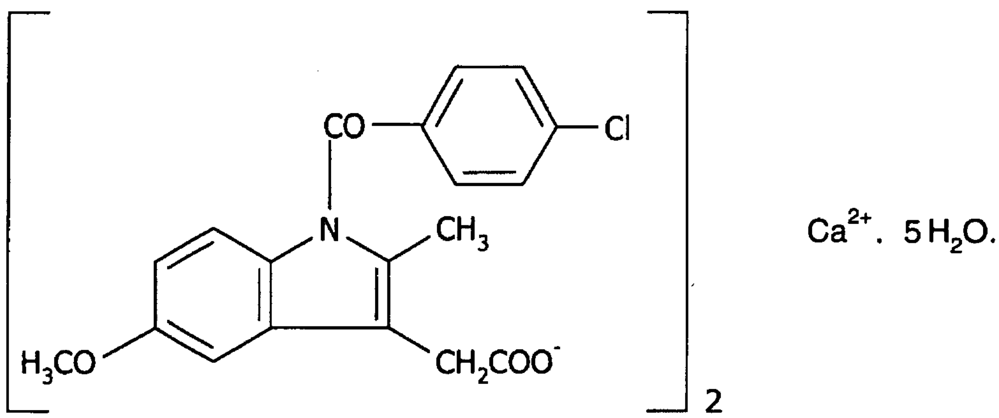

**2.** Indomethacin calcium according to claim 1, **characterised in that** it has a free indomethacin content of less than or equal to 0.5 % by weight and/or an indomethacin degradation product(s) content of less than or equal to 0.2 % by weight.

**3.** Pharmaceutical compositions, **characterised in that** they contain a pharmacologically active amount of indomethacin calcium according to one of claims 1 or 2, advantageously incorporated in a pharmaceutically acceptable excipient, support or vehicle.

**4.** The pharmaceutical compositions according to claim 3, **characterised in that** they consist of galenic preparations which are acceptable for administration via the oral route, via the rectal route, via injectable route or for local, dermal or ophthalmic applications.

**5.** A method for preparing indomethacin calcium pentahydrate, **characterised in that** it comprises :

- the reaction, in aqueous medium, of indomethacin (acid) with calcium carbonate ; said reagents, indomethacin (acid) : calcium carbonate, being incorporated in a molar proportion of between 2 :1 and 2 : 2 ; and
- drying the paste obtained after said reaction, and then keeping it in ambient air until its mass remains constant.

**6.** The method according to claim 5, **characterised in that** said reagents are incorporated in a stoichiometric molar proportion (2 : 1) or close to stoichiometry.

**7.** The method according to one of claims 5 or 6, **characterised in that** said reagents are incorporated in a molar proportion of 2 : 1.1.

**8.** The method according to any one of claims 5 to 7, **characterised in that** said reaction is carried out :

- at a temperature of less than or equal to 30°C ; and
- under conditions in which the accumulation, in the reaction medium, of the $CO_2$ formed is minimised, even stopped.

**9.** The method according to any one of claims 5 to 8, **characterised in that** said reaction is carried out at a temperature of less than 30°C.

**10.** The method according to any one of claims 5 to 9, **characterised in that** it is carried out with agitation.

**11.** The method according to claim 10, **characterised in that** it is carried out with application of an intermittent or continuous vacuum and/or introduction of the calcium carbonate into the reaction mixture in a divided manner.

**12.** The method according to any one of claims 5 to 11, **characterised in that** the proportion of water, expressed in litres, of the reaction mixture is between 2 and 10 times the weight of indomethacin (free), expressed in kilograms.

**13.** The method according to any one of claims 5 to 12, **characterised in that** the proportion of water, expressed in litres, of the reaction mixture is between 2 and 7 times the weight of indomethacin (free), expressed in kilograms.